# EUROPEAN PATENT APPLICATION

(11) **EP 1 452 179 A1**
(43) Date of publication of application: **01.09.2004**
(21) Application number: 03004184.2
(22) Date of filing: 27.02.2003
(51) Int. Cl.: A61K 31/56, A61K 31/47

(54) **Novel medicament combination of a highly potent long-lasting beta2-agonist and a corticosteroid**

(71) Applicant: CHIESI FARMACEUTICI S.p.A., I-43100 Parma (IT)
(72) Inventor: Razzetti, Roberta, 43100 Parma PR (IT); Pastore, Fiorella, 43100 Parma PR (IT)
(74) Representative: Minoja, Fabrizio, Dr.

(57) **Abstract**

The present invention relates to the use of a bronchodilator in combination with an anti-inflammatory corticosteroid or an anticholinergic atropine-like derivative for the treatment of respiratory disorders and especially asthma and chronic obstructive pulmonary disease (COPD), and to pharmaceutical compositions containing the two active ingredients. In particular, the invention relates to the use of the long-acting β₂-agonist 8-hydroxy-5-[(1R)-1-hydroxy-2-[[(1R)-2-(4-methoxyphenyl)-1-methylethyl]amino]ethyl-2(1H)-quinolinone and/or physiologically acceptable salts and/or solvates thereof in combination with a corticosteroid.

## Description

### Background of the invention

Asthma is a disease which is becoming more prevalent and is the most common disease of childhood. It can be identified by recurrent wheeze and intermittent air flow limitation. Despite many advances in its understanding, said pathology remains a poorly understood and often poorly treated disease. Previously, contraction of airway smooth muscles has been regarded as the most important feature of asthma. Recently there has been a marked change in the way asthma is managed, stemming from the fact that asthma is recognized as a chronic inflammatory disease. Uncontrolled airway inflammation may lead to mucosal damage and structural changes giving irreversible narrowing of the airways and fibrosis of the lung tissue. Therapy should therefore be aimed at controlling symptoms so that normal life is possible and at the same time provide basis for treating the underlying inflammation.

The symptoms may be controlled by first generation β₂-adrenoceptor agonists such as salbutamol, fenoterol and terbutalin or second generation ones such as formoterol and salmeterol (long-acting β₂-agonists) which overcome the disadvantage of the short duration of action particularly for patients with nocturnal asthma. Prophylactic therapy, instead, is typically provided by corticosteroids such as beclometasone dipropionate, fluticasone propionate mometasone furoate and budesonide.

Another respiratory disease whose incidence is steadily increasing throughout the world is chronic obstructive pulmonary disease (COPD). Most patients with COPD have acquired their lung disease through smoking cigarettes. Depending upon trends in tobacco smoking, it is set to rise to fifth most prevalent cause of disability, worldwide by 2020 (Leckie M et al *Exp* *Opin Invest Drugs* 2000, 9, 3-23).

Chronic obstructive pulmonary disease (COPD) is defined as a disease state characterized by the presence of airflow obstruction due to chronic bronchitis or emphysema.

Chronic bronchitis is characterized by excessive secretion of bronchial mucus, whereas emphysema denotes abnormal, permanent enlargement of air spaces distal to the terminal bronchiole, with destruction of their walls and without obvious fibrosis (American Thoracic Society). Each condition is treated as specific diseases.

Chronic obstructive bronchiolitis is due to obstruction of the peripheral airways as a result of inflammation in the bronchioles.

Drugs intended for the treatment of lung diseases such as asthma and COPD are currently administered by pulmonary delivery which relies on inhalation of an aerosol through the mouth and throat so that the drug substance can reach the lung. They can be administered as aqueous or hydroalcoholic formulations through a nebuliser, as dry powders by means of Dry Powder Inhalers or in halogenated hydrocarbon propellants. The propellant-based systems require suitable pressurized metered-dose inhalers (pMDIs) which release a metered dose of medicine upon each actuation.

Complicated therapy with different medications and devices may lead to poor compliance of the patients, so to under-treatment and, in turn, negative impact on their quality of life. This is dramatically evident in the case of long-term management of chronic asthma, in particular with prophylactic treatments, such as inhaled steroids, which do not give immediate symptom relief. Recent therapeutic strategy is aimed at both controlling the symptoms and reducing the inflammation by fixed combinations of a long-acting β₂-agonist and a corticosteroid.

Combinations containing salmeterol and fluticasone propionate, both under the form of dry powder and HFA formulations, are currently on the market under the trade name of Seretide® Each dose of the combined formulations is administered twice-daily. Each inhalation from the powder formulation delivers a nominal dose of 50 microg of *rac*-salmeterol xinafoate and one of three doses of fluticasone propionate 100, 250 and 500 microg. Using the HFA formulation, each puff from the inhaler delivers a nominal dose of 25 microg of salmeterol and 50, 125 or 250 microg of fluticasone.

A combination containing formoterol and budesonide in form of dry powder is currently on the market under the trade name of Symbicort® and each single dose is administered twice-daily. Each inhalation delivers a nominal dose of 6 microg of rac-formoterol fumarate and either 100 or 200 microg of budesonide.

Both the combinations on the market at least for the currently recommended doses are administered twice-daily. The long-acting β₂-agonist present in said combinations are under racemic form. In particular formoterol is a mixture of (R,R)- and (S,S)-enantiomers, wherein the bronchodilator activity resides in the (R,R)-one while the other one is practically inactive; salmeterol instead is a mixture of (R)- and (S)-enantiomers with the latter one much less effective than the former (Waldeck B *Gen Pharmac.1996, 27,* 575-580). Although there is no compelling evidence that the presence of the less active or inactive enantiomer is of any harm to the patient (Waldeck B Eur J Pharmacol 2002, 445, 1-12), some evidence suggest, on the other hand, that potential improvements in therapeutic indices can be achieved with isomerically pure versions of racemic drugs (Handley DA et al Curr Opin Pulm Med 2000, 6, 43-49).

Several articles in the scientific literature deal with the use of β2-agonists in combination with other classes of drugs, in particular corticosteroids and anticholinergics.

Moreover, various kinds of combinations and in particular β₂-agonists and corticosteroids combinations have been proposed in the patent literature.

Nevertheless, it has never been demonstrated in the prior art that, by using a long-acting β₂-agonist (LABA) in the combination therapy, an increment of the anti-inflammatory efficacy can be achieved so as making possible to reduce the dose without affecting the therapeutic effect.

Therefore, in view of the above considerations, it would be highly advantageous to provide a combination of a β₂-agonist and a steroid which: i) while keeping the rapid onset of action, has a longer duration of action in such a way as that the formulation can be administered once a day so delaying the possible appearance of tolerance towards the β₂-agonist and with a great improvement of the compliance of patients, in particular of those with chronic and nocturnal asthma; *ii)* allows to reduce the dose of corticosteroid.

2(1H)-quinolone derivatives have been disclosed in the past, for instance in EP 147719 and WO 00/75114, and characterized as potent long lasting bronchodilating agents useful for the treatment or prophylaxis of various chronic obstructive pulmonary disease.

A medicament comprising a particular 2(1H)-quinolinone derivative LABA and a corticosteroid for the treatment of inflammatory or obstructive airways diseases has been recently disclosed in WO 02/45703. In the description it is stated in general terms that it is possible to use said combination to reduce the dosages of corticosteroid required for a given therapeutic effect compared with those required using treatment with a corticosteroid alone, but no supporting evidence is reported.

8-Hydroxy-5-[(1R)-1-hydroxy-2-[[(1R)-2-(4-methoxyphenyl)-1-methyl ethyl] amino]ethyl-2(1H)-quinolinone hydrochloride salt, known with the experimental code TA 2005 is a highly potent long-acting β₂-agonist also characterized by a rapid onset of action, disclosed for the first time in EP 147719. In the description it is stated that the compound can be administered either orally or parenterally. As far as the daily dose is concerned, only a very broad generic range is reported, i.e. 0.01 to 30 µg, especially 0.01 to 3 µg per kg of body weight (corresponding to about 0.7 to 2100 µg, especially 0.7 to 210 µg). JP 09-309830 referred to its use as an anti-inflammatory agent by inhalation but even in this case, a broad generic range of doses was reported, i.e. from 1 to 20 µg, for example about 3 to 10 µg.

In Eur Resp J 8 (Suppl 19), 1995, 258s, the results of two clinical studies were shown. In the latter (P1300), mild asthmatics inhaled single doses of 6 and 9 µg TA 2005 and placebo from a metered dose nebuliser system at one week intervals. At these two dose levels TA 2005 produced a rapid and long lasting bronchodilation. The former (P1301) concerned a randomised, double bind, placebo-controlled, rising dose, safety study. Single doses of either 0.8, 1.6, 3.2, 6.4, 9.6, 12.8 µg were inhaled by healthy male volunteers from a metered dose nebuliser system. TA 2005 caused a dose-dependent increase in heart rate, tremor and pulmonary function and a decrease in plasma potassium. According to the authors, the maximum no adverse effect dose of TA 2005 was 9.6 µg. A very large therapeutic window between 0.8 and 9.6 µg has been therefore suggested, but an efficacious and safe dose has not been identified.

In EP 1157689 the applicant described aerosol pharmaceutical compositions comprising a β₂-agonist belonging to the class of phenylalkylamino derivatives in solution in a HFA propellant and a co-solvent whose apparent pH was adjusted to between 2.5 and 5.0 in order to guarantee an adequate shelf-life of the medicament. In the description, it has generically been stated that TA 2005 formulations will be advantageously suitable for delivering 2-10 µg/dose, preferably 3-5 µg/dose. A 3.5 µg/dose HFA 134a formulation containing 12% w/w ethanol and 1.0% IPM has been reported in example 7.

Several other patents or patent applications, i.e. US 6221398, US 5874063, WO 98/41193, WO 98/31352 and WO 01/78693 separately mention TA 2005 among other β₂-agonists and other classes of drugs such corticosteroids and anticholinergics.

In none of the documents of the prior art, a suitable therapeutic daily dose has been disclosed neither for TA 2005 as such nor for its combination with corticosteroids or other active ingredients such as anticholinergics.

### SUMMARY OF THE INVENTION

According to a first aspect, the present invention provides a medicament comprising, separately or together: 8-hydroxy-5-[(1R)-1-hydroxy-2-[[(1R)-2-(4-methoxyphenyl)-1-methylethyl]amino]ethyl-2(1H)-quinolinone (compound A), and/or a physiologically acceptable salt and/or solvate thereof and a corticosteroid useful for the treatment of the inflammatory or obstructive airways diseases preferably selected from the group of budesonide and its epimers, beclometasone dipropionate, flunisolide, fluticasone propionate, ciclesonide, triamcinolone acetonide, rofleponide palmitate and mometasone furoate (compounds B), for simultaneous, sequential or separate administration in the prophylaxis or treatment of an inflammatory or obstructive airways disease such as asthma or COPD.

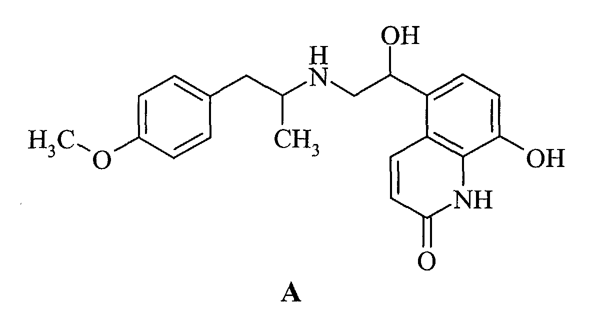

In a further aspect, the present invention provides a pharmaceutical composition comprising, together, effective amounts of (A) as hereinbefore defined and a corticosteroid, as hereinbefore defined, optionally together with at least one pharmaceutically acceptable carrier.

The invention further provides the use of (A) as hereinbefore defined in combination with a corticosteroid, as hereinbefore defined in the preparation of a pharmaceutical composition or a kit for the prophylaxis or treatment, by simultaneous, sequential or separate administration of (A) and the corticosteroid, of any inflammatory or obstructive airways disease.

It has indeed been found that, upon combination of such β₂-agonist and a corticosteroid, the anti-inflammatory effects increase.

Accordingly, it might be possible to reduce the dose of the steroid without affecting the therapeutic effect, so diminishing the risk of appearance of the side-effects associated to its use.

The synergistic increment of the anti-inflammatory effects may be attributed to the strengthening of those mediated via the β₂-adrenoreceptor, i.e. the decrease in the inflammatory cytokine tumour necrosis factor-α (TNFα) release and the increase of the anti-inflammatory cytokine interleukin-10 (IL-10), which in turn depend on the stereochemistry of the compound.

In a particular aspect, the present invention provides a medicament wherein the compound (A) is present in the combination as hydrochloride salt (TA 2005) in a suitable amount to provide a daily dose comprised between 0.5 and 8 µg, advantageously between 1 and 6 µg, preferably between 2 and 4 µg, for simultaneous, sequential or separate administration once or twice daily, preferably once daily, in the treatment of an inflammatory or obstructive airways disease such as asthma or COPD.

It has indeed been found that this is the daily dose of TA 2005 therapeutically effective and well tolerated upon inhalation and that gives rise to a higher anti-inflammatory effect in the combination with the corticosteroid.

### DETAILED DESCRIPTION OF THE INVENTION

Compound (A) is preferably used in the form of its hydrochloride salt (TA 2005).

Other suitable physiological salts of compound (A) include bromide, sulphate, phosphate, maleate, fumarate, tartrate, citrate, benzoate, mesilate, ascorbate, salicylate, acetate, succinate, lactate, glutarate or gluconate. Solvates of salts such as hydrates, emihydrates or others with pharmaceutically acceptable organic solvents are also comprised in the invention.

Among the corticosteroids, budesonide and its 22R-epimer are particularly preferred. Instead of corticosteroids, the medicament of the invention can comprise anticholinergic atropine-like derivatives such as ipratropium bromide, oxitropium bromide, tiotropium bromide, in order to provide a medicament particularly effective for the treatment of COPD.

The ratios in which the two active substances, i.e. the compound (A) and the corticosteroid, may be used in the combination of the invention are variable. Depending on the choice of the steroid, the ratios by weight which may be used within the scope of the present invention vary on the basis of the different molecular weights of the various steroids and their different potencies.

The pharmaceutical combinations according to the invention may contain compound (A) and the corticosteroid in ratios by weight ranging from 1: 3200 to 1: 10.

In particular for budesonide, flunisolide, ciclesonide and BDP, the ratio by weights ranges from 1:1600 to 1:10, preferably from 1:500 to 1:50, for fluticasone propionate from 1:1000 to 1:10, preferably from 1:500 to 1:20, for mometasone furoate from 1:800 to 1:20, preferably from 1:200 to 1:50 and for the 22R-epimer of budesonide from 1:320 to 1:20, preferably from 1:160 to 1:40.

The intended dose regimen is twice or once daily, preferably once daily, where the suitable daily dose of compound (A) is advantageously the range of 0.5 to 8 µg, preferably of 1 to 6 µg, more preferably of 2 to 4 µg and the suitable daily dose of the steroid is in the range of 10 to 2000 µg. In particular for budesonide and BDP, the suitable dose is in the range of 50 to 1600 µg, for fluticasone propionate in the range of 25 to 1000 µg, for mometasone furoate in the range of 25 to 400 µg and for the 22R-epimer of budesonide in the range of 20 to 160 µg. For both compound (A) and the steroid, the dose regimen will strongly depend on the patient (age, weight etc.) and the severity of the disease.

The combination of the invention is preferably administered by inhalation. Inhalable preparations include inhalable powders, propellant-containing metering aerosols or propellant-free inhalable formulations.

For administration as a dry powder, single- or multi-dose inhalers known the prior art wherein the powder can be filled in gelatin, plastic or other capsules, cartridges or blister packs or in a reservoir.

A diluent or carrier, generally non-toxic and chemically inert to the medicaments, e.g. lactose or any other additive suitable for improving the respirable fraction can be added to the powdered medicament.

Inhalation aerosols containing propellant gas such as hydrofluoroalkanes may contain both the active ingredient of the combination of the invention in solution or in dispersed form or one component dissolved and the other dispersed.

The propellant-driven formulations may also contain other ingredients such as co-solvents, stabilizers, surfactants, antioxidants.

The propellant-free inhalable formulations comprising the combination of the invention may be in form of solutions or suspensions in an aqueous, alcoholic or hydroalcoholic medium and they may be delivered by jet or ultrasonic nebulizers known from the prior art or by soft-mist nebulizers such as Respimat® .

The unexpected features of the combination of the present invention is demonstrated in an in vivo model of Sephadex-induced lung inflammation according to the method reported by Kublin R et al Int Arch Allergy Immunol 1992; 98: 266-272 as well as in an in vitro assay on human U937-derived macrophage cell line as described in Naunyn-Schmiedeberg's Arch. Pharmacol., 362:184-189, 2000.

Treatment of inflammatory or obstructive airways diseases in accordance with the invention may be symptomatic or prophylactic treatment. Inflammatory or obstructive airways diseases to which the claimed combinations are applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e. g. of less than 4 or 5 years of age, exhibiting wheezing symptoms and diagnosed or diagnosable as "wheezy infants", an established patient category of major medical concern. Prophylactic efficacy in the treatment of asthma will be evidenced by reduced frequency or severity of symptomatic attack, e. g. of acute asthmatic or bronchoconstrictor attack, improvement in lung function or improved airways hyperreactivity. It may further be evidenced by reduced requirement for other, symptomatic therapy. Prophylactic benefit in asthma may in particular be apparent in subjects prone to "morning dipping". "Morning dipping" is a recognized asthmatic syndrome, common to a substantial percentage of asthmatics and characterized by asthma attack, e. g. between the hours of about 4 to 6 a.m., i. e. at a time normally substantially distant form any previously administered symptomatic asthma therapy.

Other inflammatory or obstructive airways diseases and conditions to which the present invention is applicable include acute lung injury (ALI), adult respiratory distress syndrome (ARDS), chronic obstructive pulmonary, airways or lung disease (COPD, COAD or COLD) including chronic bronchitis and emphysema, bronchiolitis, bronchiectasis and exacerbation of airways hyperreactivity consequent to other drug therapy, in particular other inhaled drug therapy.

Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tobacosis and byssinosis.

The invention further provides a pharmaceutical kit comprising compound (A) and a corticosteroid, as hereinbefore described, in separate unit dosage forms, said forms being suitable for administration of (A) and the corticosteroid in effective amounts. Such a kit suitably further comprises one or more inhalation devices for administration of (A) and the corticosteroid. For example, the kit may comprise one or more dry powder inhalation devices adapted to deliver dry powder from a capsule, together with capsules containing a dry powder comprising a dosage unit of (A) and capsules containing a dry powder comprising a dosage unit of the corticosteroid. In another example, the kit may comprise a multidose dry powder inhalation device containing in the reservoir thereof a dry powder comprising (A) and a multidose dry powder inhalation device containing in the reservoir thereof a dry powder comprising the corticosteroid. In a further example, the kit may comprise a metered dose inhaler containing an aerosol comprising (A) in a propellant and a metered dose inhaler containing an aerosol comprising the corticosteroid in a propellant.

The invention is illustrated with reference to the following examples.

### Example 1 - In vitro assay on human U-937 derived macrophage cell line of the anti-inflammatory efficacy of the combination of the invention

U-937 cells are cultured and macrophage-differentiated by 10 ng/ml phorbol myristate acetate for 48 hours.

The cells are then incubated with 1 µg/ml lipopolysaccharide (LPS) or LPS together with concentration ranges of β₂-agonist, corticosteroid, or combination. After 30 minutes the medium is collected for TNFα and IL-10 and the cells lysed with lysis buffer for cyclic adenosine 3',5'-monophosphate (cAMP) measurement.

TNFα and IL-10 in the culture medium are determined by a commercial available ELISA kit, while cAMP is determined by a commercial ³H-cAMP analysis kit.

TA 2005 is used in concentration ranges of 10⁻¹¹ to 3x10⁻⁶ M while three different concentrations of a corticosteroid is used.

### Example 2 - In vivo assay of the anti-inflammatory efficacy of the combination of the invention in a Sephadex induced lung oedema model

The Sephadex model of lung oedema in the rat is a model which leads to inflammatory cell infiltration and interstitial oedema.

Male rats (225-250 g) are housed for one week before initiating experiments. Food and water are supplied *at libitum.*

Rats are dosed intratracheally with vehicle or Sephadex beads (5 mg/ml) at a dose volume of 1 ml/kg under anesthesia.

TA 2005 and a corticosteroid are dissolved/suspended in distilled water and administered intratracheally suitably diluted in the Sephadex suspension.

Rats are killed 24 h post-administration, the lungs are removed and the lung wet weights determined. Percent inhibition of the Sephadex-induced oedema is then determined.

A greater anti-inflammatory response has been observed with the combination in comparison to either TA 2005 and corticosteroid alone.

### Example 3 - Formulation for metered dose inhaler comprising TA 2005 and 22R-budesonide

A HFA formulation for metered dose inhaler was prepared with the composition as follows:

| *Components* | *Amounts* | | |
|---|---|---|---|
| | Per unit | | Nominal dose |
| | mg | % | µg |
| TA 2005 | 0.15 | 0.0016 w/v | 1 |
| 22R-budesonide | 12.00 | 0.127 w/v | 80 |
| Ethanol | 1650.0 | 15 w/w | - |
| HC1 0.1 M | 3.30 | 0.03 w/w | - |
| Water | 220.05 | 2.0 w/w | |
| HFA 134a q.s. to 9.45 ml | 9114.5 | - | - |

The formulation (120 actuations/canister, overage of 30 actuations) was filled in aluminum canisters having the internal surface coated with Teflon (two stage pressure filling) and fitted with a metering valve having a 63 µl metering chamber.

### Example 4 - Powder formulation for dry powder inhaler comprising TA 2005 and 22R-budesonide

A powder formulation for dry powder inhaler was prepared with the composition as follows:

| *Components* | *Amounts* | |
|---|---|---|
| | Per unit dose | |
| | mg | % |
| TA 2005 | 0.001 | |
| 22R-budesonide | 0.160 | |
| Alpha-lactose monohydrate 212-355 µm | 8.8551 | 88.6 |
| Pre-blend | 0.9839 | 10 |
| | | |
| Total weight | 10 | |

### Preparation of the formulation

The pre-blend mixture was obtained as follows: alpha-lactose monohydrate SpheroLac 100 (Meggle EP D30) with a starting particle size of 50 to 400 µm and magnesium stearate with a starting particle size of 3 to 35 µm in the ratio 98:2 w/w were co-milled in a jet mill apparatus.

About 88% w/w alpha-lactose monohydrate CapsuLac (212 ― 355 µm) was placed in a 2.5 l stainless steel container, then 10% w/w of the pre-blend mixture was added. The powder was mixed in a Turbula mixer for 4 hours at 32 r.p.m. TA 2005 and 22R-budesonide were added to the powder and mixed in a Turbula mixer to obtain, respectively, a ratio of 1 µm and 160 mg of the active ingredients to 10 mg of carrier.

## Claims

1. A combined preparation comprising:
- 8-Hydroxy-5-[(1R)-1-hydroxy-2-[[(1R)-2-(4-methoxyphenyl)-1-methylethyl]amino]ethyl-2(1H)-quinolinone (compound A) and/or a physiologically acceptable salt and/or solvate thereof
- a corticosteroid useful for the treatment of the inflammatory or obstructive airways diseases,
for simultaneous, sequential or separate use in the treatment of an inflammatory or obstructive airways disease.

2. A preparation according to claim 1 wherein the compound (A) is in the form of hydrochloride salt.

3. A preparation according to claim 2 wherein the daily dose of the compound (A) is the range of 0.5 to 8 µg.

4. A preparation according to claim 3 wherein the daily dose is in the range of 1 to 6 µg.

5. A preparation according to claim 3 wherein the daily dose is in the range of 2 to 4 µg.

6. A preparation according to claims 1-5 wherein the corticosteroid is selected from budesonide and its epimers, beclometasone dipropionate, fluticasone propionate, flunisolide, ciclesonide, triamcinolone acetonide, rofleponide palmitate and mometasone furoate.

7. A preparation according to claim 6 wherein the corticosteroid is budesonide or its epimer 22R

8. A preparation according to claim 5 wherein the ratio by weight between the compound (A) and the corticosteroid is from 1: 3200 to 1: 10.

9. An inhalatory pharmaceutical composition comprising an effective amount of the compound (A) and a of a corticosteroid, together with at least one pharmaceutically acceptable carrier.

10. A pharmaceutical composition according to claim 9 to be administered by pressurized metered dose inhalers wherein the active ingredients are in solution or suspension in a propellant gas.

11. A pharmaceutical composition according to claim 9 to be administered by a nebulizer comprising a solution or a suspension of the compound (A) and a corticosteroid in an aqueous, alcoholic or hydroalcoholic medium.

12. A pharmaceutical composition according to claim 9 to be administered by a dry powder inhaler in which the compound (A) and the corticosteroid are in dry powder form.

13. A pharmaceutical kit comprising the compound (A) and a corticosteroid in separate unit dosage forms, said forms being suitable for administration of (A) and the corticosteroid in effective amounts, together with one or more inhalation devices for administration of (A) and of the corticosteroid.

14. A combined preparation comprising:
- the compound (A) and/or a physiologically acceptable salt and/or solvate thereof and
- a anticholinergic atropine-like derivative selected form the group of ipratropium bromide, oxitropium bromide, tiotropium bromide
for simultaneous, sequential or separate use in the treatment of an inflammatory or obstructive airways disease.

15. An inhalatory pharmaceutical composition comprising an effective amount of the compound (A) and of an anticholinergic atropine-like derivative selected form the group of ipratropium bromide, oxitropium bromide, tiotropium bromide, together with at least one pharmaceutically acceptable carrier.
